# EUROPÄISCHE PATENTSCHRIFT

(11) **EP 4 216 897 B1**
(45) Veröffentlichungstag und Bekanntmachung des Hinweises auf die Patenterteilung: **23.09.2026**
(21) Anmeldenummer: 20838332.3
(22) Anmeldetag: 23.12.2020
(51) Int. Cl.: A61H 3/06, G02C 11/00, G02C 11/04, G02C 5/14, G01S 17/93, G02C 5/00, A61F 9/08, G01S 17/08, G01S 17/87, G01S 17/88

(54) **LEITVORRICHTUNG FÜR PERSONEN MIT EINGESCHRÄNKTEM SEHVERMÖGEN**
GUIDE APPARATUS FOR PERSONS WITH IMPAIRED VISION
APPAREIL DE GUIDAGE POUR PERSONNES SOUFFRANT DE DÉFICIENCE VISUELLE

(30) Priorität: 22.09.2020 AT 508052020
(43) Veröffentlichungstag der Anmeldung: 02.08.2023
(73) Patentinhaber: Scheu, Thomas, 7311 Neckenmarkt (AT); Holzinger, Tobias, 2301 Groß-Enzersdorf (AT)
(72) Erfinder: Scheu, Thomas, 7311 Neckenmarkt (AT); Holzinger, Tobias, 2301 Groß-Enzersdorf (AT)
(74) Vertreter: Wildhack & Jellinek Patentanwälte GmbH
(86) Internationale Anmeldenummer: PCT/AT2020/060486
(87) Internationale Veröffentlichungsnummer: WO 2022/061380

(56) Entgegenhaltungen:
- US-B1- 10 576 011
- KOCHAKARN PAWIT: "SonicScape (Binaural Sensor Glasses for the Blind) | Hackaday.io", 1 January 2017 (2017-01-01), XP055810000, Retrieved from the Internet <URL:https://hackaday.io/project/20899-sonicscape-binaural-sensor-glasses-for-the-blind> [retrieved on 20210602]
- AMFT OLIVER ET AL: "Making Regular Eyeglasses Smart", IEEE PERVASIVE COMPUTING, IEEE SERVICE CENTER, LOS ALAMITOS, CA, US, vol. 14, no. 3, 1 July 2015 (2015-07-01), pages 32 - 43, XP011662334, ISSN: 1536-1268, [retrieved on 20150630], DOI: 10.1109/MPRV.2015.60
- VIAVI SOLUTIONS: "Optical Filters for LiDAR Systems", 19 June 2020 (2020-06-19), XP055809086, Retrieved from the Internet <URL:https://www.viavisolutions.com/en-us/literature/optical-filters-lidar-systems-white-papers-books-en.pdf> [retrieved on 20210531]
- WONG BRIAN: "Jumping Hurdles To Build A Better LiDAR | FierceElectronics", 15 January 2019 (2019-01-15), XP055809323, Retrieved from the Internet <URL:https://www.fierceelectronics.com/components/jumping-hurdles-to-build-a-better-lidar> [retrieved on 20210531]

## Beschreibung

Die Erfindung betrifft eine Leitvorrichtung für Personen mit eingeschränktem Sehvermögen gemäß Patentanspruch 1.

Aus dem Stand der Technik ist eine Vielzahl von Vorrichtungen bekannt, die von blinden Personen oder Personen mit beeinträchtigtem Sehvermögen mitgeführt werden können, sodass sich diese Personen selbstständig auch in einer ihnen fremden Umgebung orientieren bzw. bewegen können. Beispielsweise können von blinden Personen bzw. Personen mit beeinträchtigtem Sehvermögen Blinden- oder Langstöcke mitgeführt werden, die speziell im städtischen Raum, wo auf der Straße und in öffentlichen Einrichtungen Orientierungshilfen auf dem Boden verbaut sind, für die Orientierung genutzt werden können.

Sind derartige Einrichtungen nicht vorhanden, können beispielsweise Vorrichtungen, die in Headsets oder brillenartige Halterungen integriert sind und die Abstände zu Hindernissen detektieren, von der Person getragen werden, um sich in der Umgebung zu orientieren. Derartige Vorrichtungen sind z.B. aus der US 10,576,011 B1, der US 2019/070064 A1, der DE 202012006247 U1, der WO 2015/121846 A1, oder der WO 9701320 A1 bekannt.

Derartige Vorrichtungen können auch in Halsbändern bzw. Halsketten oder Kleidungsstücken integriert sein, wie sie z.B. aus der US 2017/032787 A1, der US 2015/201181 A1, der US 2015/198455 A1, der GB 2538445 A, der US 9311827 B1, der US 2016/284235 A1 oder der CH 709806 A1 bekannt sind.

Nachteil bei den bekannten Vorrichtungen ist jedoch, dass diese einerseits gar keine oder nur eine schlechte räumliche Auflösung des Abstands zu Hindernissen bieten, die verwendeten Sensoren oftmals von Fremdeinflüssen gestört werden, und die Vorrichtungen oftmals sperrig und schwer sind, sodass sie keinen hohen Tragekomfort bieten und nicht über einen längeren Zeitraum getragen werden können. Weiters sind die bekannten Vorrichtungen zumeist auch unmittelbar als Hilfseinrichtung für Blinde bzw. sehbeeinträchtigte Personen erkennbar, was vom Träger der Vorrichtung oft nicht erwünscht ist.

Aufgabe der Erfindung ist es daher, eine Leitvorrichtung für Personen mit beeinträchtigtem Sehvermögen bereitzustellen, die einerseits eine gute räumliche Auflösung der Umgebung, insbesondere des Abstands zu Hindernissen, gewährleistet, zuverlässig und gleichzeitig kompakt ausgebildet, bequem über einen langen Zeitraum zu tragen, sowie nicht unmittelbar als Hilfsvorrichtung erkennbar ist.

Die Erfindung löst diese Aufgabe mit einer Leitvorrichtung für Personen mit eingeschränktem Sehvermögen gemäß Patentanspruch 1. Erfindungsgemäß ist dabei vorgesehen, dass die Leitvorrichtung folgende Komponenten umfasst:
Eine Brille, zwei Lidar-Sensoren, zwei Aktuator-Einheiten und eine mit den Lidar-Sensoren und den Aktuator-Einheiten verbundene Steuer- und Verarbeitungseinheit,
- wobei die Lidar-Sensoren an der Frontseite der Brille, insbesondere am Brillenrahmen, angeordnet sind und zur Erfassung der Umgebung des jeweiligen Lidar-Sensors, insbesondere des Abstands zu Hindernissen, ausgebildet sind, wobei ein Filter zur Reduktion des Einflusses von Störlicht auf die Messwerte der Lidar-Sensoren vorgesehen ist,
- wobei die Steuer- und Verarbeitungseinheit an der, dem Kopf einer Person zugewandten, Innenseite der Brille, insbesondere am Brillenrahmen oder an einem Brillenbügel, angeordnet ist und dazu ausgebildet ist, Abstandsmessungen mittels der Lidar-Sensoren durchzuführen und die ermittelten Abstandsmesswerte in Steuersignale für die Aktuator-Einheiten umzuwandeln und an die Aktuator-Einheiten zu übermitteln, und
- wobei jede Aktuator-Einheit jeweils an der Innenseite eines Brillenbügels angeordnet ist und dazu ausgebildet ist, zur Darstellung der Abstandsmesswerte für Personen wahrnehmbare Signale abzugeben.

Durch diese Ausgestaltung einer erfindungsgemäßen Leitvorrichtung ist es einerseits vorteilhaft möglich, durch den Einsatz von zwei Lidar-Sensoren an der Frontseite der Brille eine gute räumliche Auflösung der Umgebung bzw. der Position von Hindernissen in der Umgebung der Person zu erzielen. Weiters ist es durch diese Ausgestaltung einer erfindungsgemäßen Leitvorrichtung möglich, dass die Person die Leitvorrichtung auch ganztätig gebraucht, ohne dass diese beispielsweise durch hohes Gewicht eine Einschränkung bzw. Belastung für die Person darstellt. Dadurch, dass die Steuer- und Verarbeitungseinheit die Abstandsmesswerte im Steuersignal für die Aktuator-Einheiten umwandelt, ist es vorteilhafterweise möglich, dass die Person zuverlässig vor Hindernissen gewarnt wird, an die sie sich annähert, sodass sich die Person zuverlässig und sicher in einer für sie fremden Umgebung bewegen kann.

Unter einer Leitvorrichtung wird im Folgenden eine Vorrichtung verstanden, die blinden Personen bzw. Personen mit beeinträchtigtem Sehvermögen dabei hilft, sich in ihrer Umgebung sicher fortzubewegen, indem sie an die Person Signale abgibt, die ihr dabei helfen, Hindernisse, die sich in ihrer Umgebung befinden, zu umgehen.

Unter Lidar-Sensoren - die Abkürzung "Lidar" steht für *light detection and ranging -* werden im Folgenden Sensoren verstanden, die Laserstrahlen aussenden und das von Objekten, auf die die Laserstrahlen auftreffen, reflektierte Licht detektieren und daraus Messwerte für eine Weiterverarbeitung generieren. Die Messwerte können z.B. dazu genutzt werden, die Entfernung der Objekte zu bestimmen, die die Laserstrahlen reflektiert haben.

Unter einem Filter werden im Folgenden alle Vorrichtungen und rechnerische Vorgehensweisen verstanden, die dazu geeignet sind, den Einfluss von Störlicht auf die Messwerte der Lidar-Sensoren zu reduzieren. Dabei kann es sich beispielsweise um einen physikalischen Filter handeln, der bestimmte Wellenlängen am Passieren zum jeweiligen Lidar-Sensor hindert, oder einen rechnerischen Filter, der z.B. bestimmte Wellenlängen aus dem vom Lidar-Sensor empfangenen Wellenlängenspektrum entfernt.

Unter Aktuator-Einheiten werden alle Vorrichtungen verstanden, die dazu geeignet sind, ermittelte Messwerte in einer für Personen, insbesondere für Personen mit eingeschränktem Sehvermögen, wahrnehmbaren Form, z.B. in Form von taktilen oder akustischen Signalen, abzugeben.

Eine weitere Verbesserung bei der räumlichen Auflösung von detektierten Abstandsmesswerten durch eine besonders effektive Reduktion von Störlichteinflüssen kann erzielt werden, wenn vor jedem der Lidar-Sensoren jeweils ein optischer Filter angeordnet ist, wobei der optische Filter dazu ausgebildet ist, Licht mit einer Wellenlänge von 780 nm bis 1 mm, insbesondere 780 nm bis 3 µm, vorzugsweise 940 nm, passieren zu lassen.

Eine weitere besonders effektive Minimierung von Störlichteinflüssen auf die Sensormesswerte und somit die ermittelten Abstandsmesswerte kann erzielt werden, wenn die Steuer- und Verarbeitungseinheit dazu ausgebildet ist, ausgewählte, vorzugsweise alle, ermittelten Abstandsmesswerte jeweils einem digitalen Filter zur Reduktion des Einflusses von Störlicht zuzuführen und die derartigen durch Filterung ermittelten gefilterten Abstandsmesswerte für die Umwandlung in Steuersignale heranzuziehen.

Eine weitere Verbesserung der räumlichen Auflösung des detektierten Abstands zu Hindernissen in der Umgebung der Person kann erzielt werden,
- wenn ein Lidar-Sensor rechts und ein weiterer Lidar-Sensor links jeweils an der Frontseite der Brille, insbesondere am Brillenrahmen, angeordnet ist,
- wenn eine Aktuator-Einheit am linken Brillenbügel und eine weitere Aktuator-Einheit am rechten Brillenbügel angeordnet ist.

Eine für den Träger der Leitvorrichtung besonders zuverlässige Warnung vor Hindernissen, an die er sich annähert, kann erzielt werden, wenn die Steuer- und Verarbeitungseinheit dazu ausgebildet ist, die vom linken Lidar-Sensor stammenden Abstandsmesswerte in Steuersignale für die linke Aktuator-Einheit umzuwandeln und die vom rechten Lidar-Sensor stammenden Abstandsmesswerte in Steuersignale für die rechte Aktuator-Einheit umzuwandeln.

Eine vorteilhafte Ausgestaltungsvariante einer Leitvorrichtung, mit der sich blinde und sehbehinderte Personen besonders einfach in ihrer Umgebung orientieren können, sieht vor, dass die Steuer- und Verarbeitungseinheit dazu ausgebildet ist, die Intensität des von der jeweiligen Aktuator-Einheit abzugebenden Signals in Abhängigkeit vom ermittelten Abstandsmesswert vorzugeben, insbesondere die Intensität des Signals bei sich verringerndem Abstand zu erhöhen.

Um für die Person, die die Leitvorrichtung trägt, die Wahrnehmung von Entfernungen bzw. Abständen zu Hindernissen weiter zu vereinfachen, kann vorgesehen sein, dass die Steuer- und Verarbeitungseinheit dazu ausgebildet ist, den jeweils ermittelten Abstandsmesswert mit zumindest einem, insbesondere vorab vorgegebenen und/oder einstellbaren, Abstandsschwellenwert zu vergleichen und auf Grundlage des Vergleichs die Intensität des von der jeweiligen Aktuator-Einheit abzugebenden Signals vorzugeben.

Eine Möglichkeit, gemäß der Erfindung, die Distanz einzustellen, ab der die Person, die die Leitvorrichtung trägt, gewarnt wird, um so eine Anpassung der Warnung an verschiedene Umfelder zu ermöglichen, kann erzielt werden,
- wenn die Leitvorrichtung zumindest einen, insbesondere am Brillenbügel angeordneten, mit der Steuer- und Verarbeitungseinheit verbundenen, Schalter umfasst, wobei der Schalter zumindest eine, mit einem, insbesondere in der Steuer- und Verarbeitungseinheit hinterlegten, Abstandsschwellenwert assoziierte, Schaltposition, vorzugsweise mehrere Schaltpositionen, aufweist und
- dass die Steuer- und Verarbeitungseinheit dazu ausgebildet ist, bei Verstellen des Schalters in eine Schaltposition den mit der jeweiligen Schaltposition assoziierten Abstandsschwellenwert für die Umwandlung der ermittelten Abstandsmesswerte in Steuersignale für die Aktuator-Einheiten, insbesondere für die Vorgabe der Intensität des von der jeweiligen Aktuator-Einheit abzugebenden Signals, heranzuziehen.

Auf diese Weise ist es möglich, dass die Person je nach Umfeld, d.h. je nachdem ob sie sich in einer Stadt, in der sich in kürzerer Distanz eine Vielzahl von Hindernissen befindet, oder z.B. auf einer Wiese oder einem Park, wo Hindernisse unter Umständen nur selten vorkommen, befindet, einstellen kann.

Eine automatisierte Anpassung der Warnung an die jeweils aktuellen räumlichen Gegebenheiten des Umfelds, in dem sich die Person, die die Leitvorrichtung trägt, befindet, kann erzielt werden, wenn die Steuer- und Verarbeitungseinheit dazu ausgebildet ist, aus einer Anzahl vorgegebener Abstandsschwellenwerte den jeweils aktuellen Abstandsschwellenwert auf den nächst niedrigeren Abstandsschwellenwert zu reduzieren, wenn die Anzahl der ermittelten Abstandsmesswerte, die den aktuellen Abstandsschwellenwert unterschreiten, einen vorgegebenen Anzahlschwellenwert, insbesondere innerhalb einer vorgegebenen Zeitspanne bzw. pro vorgegebener Zeiteinheit, überschreitet.

Konstruktiv besonders kompakt können die Aktuator-Einheiten ausgestaltet werden, wenn die Aktuator-Einheiten als Akustikelemente, insbesondere in Form von Gehörknochen-Lautsprechern, und/oder taktile Elemente ausgebildet sind.

Im Folgenden wird unter einem Akustikelement ein Element bzw. eine Vorrichtung verstanden, die dazu ausgebildet ist, für Menschen wahrnehmbare, konkret hörbare, auditive Stimuli, wie z.B. Geräusche oder Töne abzugeben. Im Folgenden wird unter einem taktilen Element ein Element bzw. eine Vorrichtung verstanden, die dazu ausgebildet ist, für Menschen wahrnehmbare, konkret fühlbare, taktile, insbesondere vibrotaktile Stimuli, abzugeben. Beispielsweise kann es sich bei einem taktilen Element um eine Vorrichtung handeln, die mit einer entsprechend von der Steuer- und Verarbeitungseinheit gesteuerten Frequenz und/oder Intensität vibriert, oder um eine Vorrichtung mit einem Fortsatz, der mit einer entsprechend von der Steuer- und Verarbeitungseinheit gesteuerten Frequenz und/oder Intensität z.B. gegen die Kopfhaut des Trägers gedrückt wird. Bei einer erfindungsgemäßen Aktuator-Einheit kann es sich jedoch auch um ein kombiniertes Element handeln, das sowohl akustische, als auch taktile Reize an die Person abgeben kann. Optional können auch sowohl Akustikelemente, als auch taktile Elemente an der Leitvorrichtung angeordnet sein.

Durch all diese Ausgestaltungen einer erfindungsgemäßen Leitvorrichtung ist gleichzeitig sichergestellt, dass der Träger der Leitvorrichtung die Signale der Aktuator-Einheiten, die die ermittelten Abstandsmesswerte für den Träger darstellen, besonders zuverlässig auch z.B. in einer Umgebung mit lauten Hintergrundgeräuschen wahrnehmen kann.

Eine besonders effektive Energieversorgung kann bei einer erfindungsgemäßen Leitvorrichtung sichergestellt werden, wenn die Leitvorrichtung eine an der, dem Kopf einer Person zugewandten, Innenseite der Brille, insbesondere am Brillenrahmen oder an einem Brillenbügel, angeordnete Energieversorgungseinheit umfasst.

Gemäß einer vorteilhaften Ausführungsform der Erfindung kann vorgesehen sein, dass die Energieversorgungseinheit Solarpaneele umfasst, die an der Frontseite der Brille, insbesondere an zumindest einem Brillenglas angeordnet sind. Mit derartigen Solarpaneelen kann die Laufzeit der Energieversorgungseinheit, die in diesem Fall bevorzugt als Akku ausgebildet ist, vorteilhafterweise deutlich erhöht werden, da zumindest eine teilweise Wiederaufladung bei Sonneneinstrahlung erzielt werden kann.

In diesem Zusammenhang kann die Person, die die Leitvorrichtung trägt, besonders zuverlässig davor gewarnt werden, dass die Leitvorrichtung aufgrund eines niedrigen Ladestands der Energieversorgungseinheit funktionsuntüchtig werden könnte, wenn die Steuer- und Verarbeitungseinheit dazu ausgebildet ist, den Ladestand der Energieversorgungseinheit, insbesondere laufend und/oder in vorgegebenen Zeitabständen, zu überprüfen und, im Fall, dass der Ladestand einen vorgegebenen Ladestand-Schwellenwert unterschreitet, ein diesbezügliches Steuersignal für zumindest eine Aktuator-Einheit zu erstellen und an die Aktuator-Einheiten zu übermitteln.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben.

Im Folgenden zeigen:
Fig. 1 eine erste Ansicht einer erfindungsgemäßen Leitvorrichtung,
Fig. 2 eine zweite Ansicht der erfindungsgemäßen Leitvorrichtung aus Fig. 1,
Fig. 3 ein zweites Ausführungsbeispiel einer erfindungsgemäßen Leitvorrichtung,
Fig. 4 eine zweite Ansicht der Leitvorrichtung aus Fig. 3,
Fig. 5 eine schematische Darstellung für die Detektion von Hindernissen mit einer erfindungsgemäßen Leitvorrichtung.

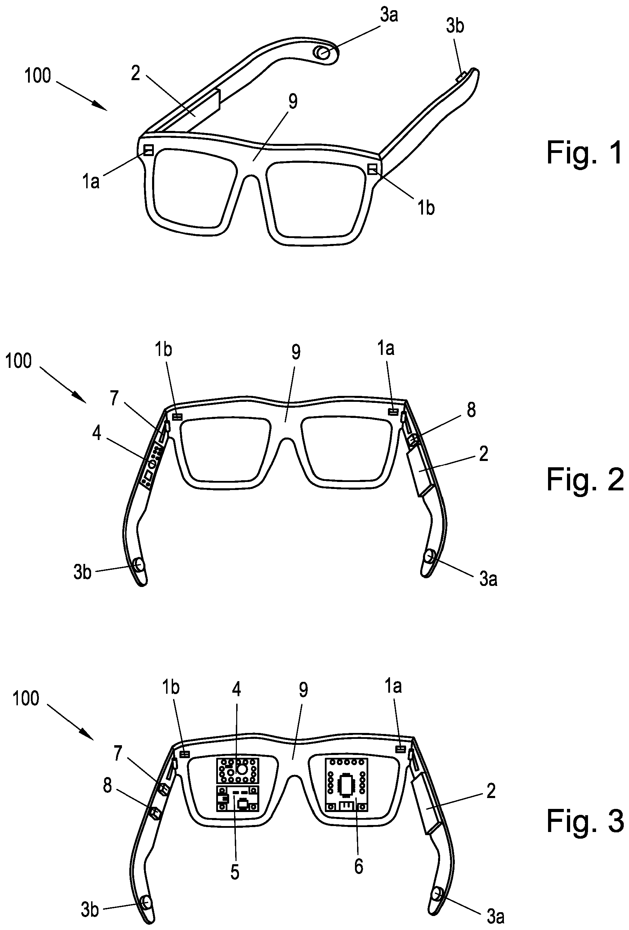

Im ersten und im zweiten Ausführungsbeispiel einer erfindungsgemäßen Leitvorrichtung 100, die in den Fig. 1 bis 4 im Detail dargestellt sind, umfasst die Leitvorrichtung 100 eine Brille 9, bei der es sich um eine Sonnenbrille mit verdunkelten Brillengläsern handelt sowie zwei Lidar-Sensoren 1a, 1b, die an der Frontseite der Brille 9 am Brillenrahmen angeordnet sind. Optional können jedoch auch mehr als zwei Lidar-Sensoren an der Brille 9 angeordnet sein.

Es ist optional auch möglich, dass die Leitvorrichtung 100 mehr als zwei, d.h. zusätzliche Sensor-Einheiten umfasst. Beispielsweise können mehrere zusätzliche Lidar-Sensoren oder andere geeignete Sensoren zur Abstandsmessung wie Ultraschallsensoren an jedem Brillenbügel, oder am Brillenrahmen, z.B. im Stirnbereich, angeordnet sein.

Die Abkürzung "Lidar" steht für *light detection and ranging* und bezeichnet ein Verfahren, bei dem Laserstrahlen ausgesandt und das von Objekten, auf die die Laserstrahlen auftreffen, reflektierte Licht detektiert werden. Aus der Lichtlaufzeit der Signale wird die Entfernung zum Ort bzw. zum Objekt berechnet, an dem die Streuung aufgetreten ist, d.h. an dem das Laserlicht gestreut wurde.

Die Lidar-Sensoren 1a, 1b der Leitvorrichtung 100 erfassen also die Umgebung des jeweiligen Lidar-Sensors 1a, 1b bzw. der Leitvorrichtung 100 und somit auch der Person, die die Leitvorrichtung 100 trägt, und detektieren Abstände zu Objekten bzw. Hindernissen, auf die sich die Person zubewegt.

Wie in den Fig. 1 bis 4 weiters ersichtlich ist, umfasst die Leitvorrichtung 100 im ersten und im zweiten Ausführungsbeispiel jeweils zwei Aktuator-Einheiten 3a, 3b, die jeweils an der Innenseite eines Brillenbügels der Brille 9, z.B. im Bereich der Ohren, angeordnet sind. In den Fig. 1 bis 5 ist eine erste Aktuator-Einheit 3a am rechten Brillenbügel angeordnet und eine zweite Aktuator-Einheit 3b am linken Brillenbügel. Es ist optional jedoch auch möglich, dass die Leitvorrichtung 100 auch mehr als zwei Aktuator-Einheiten umfasst, beispielsweise mehrere Aktuator-Einheiten an jedem Brillenbügel, oder zusätzliche Aktuator-Einheiten an der Innenseite des Brillenrahmen, z.B. im Stirnbereich.

Die Leitvorrichtung 100 umfasst weiters eine Steuer- und Verarbeitungseinheit 4, die mit den Lidar-Sensoren 1a, 1b und den Aktuator-Einheiten 3a, 3b verbunden ist. Die Steuereinheit 4 ist ebenso an der dem Kopf der Person zugewandten Innenseite der Brille 9 angeordnet. Beim ersten Ausführungsbeispiel ist die Steuer- und Verarbeitungseinheit 4 konkret am linken Brillenbügel angeordnet während im zweiten Ausführungsbeispiel die Steuer- und Verarbeitungseinheit 4 am bzw. hinter dem linken Brillenglas, beispielsweise einem getönten Brillenglas einer Sonnenbrille, angeordnet ist.

Die Steuer- und Verarbeitungseinheit 4 steuert die Lidar-Sensoren 1a, 1b an, um Abstandsmessungen durchzuführen, empfängt die von den Lidar-Sensoren 1a, 1b ermittelten Messwerte und erstellt auf Grundlage der ermittelten Abstandsmesswerte Steuersignale für die Aktuator-Einheiten 3a, 3b und übermittelt die Steuersignale an die Aktuator-Einheiten 3a, 3b. Die Aktuator-Einheiten 3a, 3b stellen anschließend die Abstandsmesswerte in Form von für Personen wahrnehmbaren Signalen dar.

Dies bedeutet, vereinfacht gesprochen, dass die Lidar-Sensoren 1a, 1b an der Vorderseite der Brille 9 die Umgebung vor dem Benutzer nach Hindernissen abtasten. Durch akustisches oder haptisches Feedback "fühlt" der Träger seine Umgebung und kann sich somit orientieren und bekommt einen räumlichen Eindruck seiner Umgebung und wird rechtzeitig vor Gefahren gewarnt wird.

Im ersten und im zweiten Ausführungsbeispiel wird der Benutzer bzw. Träger der Leitvorrichtung 100, d.h. die Person, die die Leitvorrichtung 100 trägt, über zwei Aktuator-Einheiten 3a, 3b, die jeweils hinter den Ohren auf den beiden Brillenbügeln an der Innenseite des jeweiligen Brillenbügels angeordnet sind, vor Hindernissen gewarnt. Im ersten und im zweiten Ausführungsbeispiel sind die Aktuator-Einheiten 3a, 3b als Vibrationselemente bzw. Vibrationsmotoren ausgebildet.

Alternativ kann es sich bei den Aktuator-Einheiten auch beispielsweise um Akustikelemente bzw. Akustikgeber wie Gehörknochenlautsprecher handeln. Hier können verschiedenste akustische Ausgabesignale wie Stimmausgabe, Tonmuster etc. für die Darstellung der Abstandsmesswerte für die Person bzw. den Träger eingesetzt werden.

Eine erfindungsgemäße Leitvorrichtung 100 umfasst weiters einen Filter, der den Einfluss von Störlicht auf die Messwerte der Lidar-Sensoren 1a, 1b reduziert.

Dabei kann es sich um einen optischen Filter handeln, der Licht mit einer Wellenlänge z.B. im Infrarotbereich passieren lässt und somit der Einfluss fremder Lichtquellen wie beispielsweise künstlichem Licht aus Beleuchtungen oder natürlichem Licht von der Sonne minimiert wird. Werden Lidar-Sensoren 1a, 1b eingesetzt, die Licht mit einer Wellenlänge von z.B. 940 nm detektieren, ist durch einen solchen optischen Filter, der als schmaler Infrarotbandpassfilter ausgebildet ist, eine besonders zuverlässige Detektion von Licht, das von Objekten bzw. Hindernissen in der Umgebung der Person, die die Leitvorrichtung 100 trägt, reflektiert wird, gewährleistet.

Zusätzlich oder alternativ zu einem optischen Filter kann der Filter auch ein digitaler Filter sein, der von der Steuer- und Verarbeitungseinheit 4 angewendet wird. Die Steuer- und Verarbeitungseinheit 4 kann in diesem Fall softwarebasiert zwischen Störlicht, das die Messwerte des jeweiligen Lidar-Sensors 1a, 1b beeinflusst, und dem tatsächlich von der Reflektion des ausgestrahlten Laserlichts von Oberflächen bzw. Hindernissen in der Umgebung der Person stammenden Licht, unterscheiden. Hier können beispielsweise diejenigen Wellenlängenbereiche in der Steuer- und Verarbeitungseinheit 4 hinterlegt sein, die das Nutzsignal des jeweiligen Lidar-Sensors 1a, 1b darstellen, also z.B. einen Wellenlängenbereich im Infrarotspektrum, sodass dieser Wellenlängenberich weiterverarbeitet wird, während andere Wellenlängenbereiche, die am Lidar-Sensor 1a, 1b eintreffen, verworfen werden.

Die oben bereits erwähnten, optionalen zusätzlichen Lidar-Sensoren oder andere zusätzliche Sensoren können auch die Möglichkeiten des digitalen Filters erweitern, da dadurch mehrere Messwerte zur Auswertung vorhanden sind und dadurch auf besonders effiziente Weise abgeglichen werden kann, ob ein mögliches Hindernis tatsächlich vorhanden ist.

Optional ist es in diesem Zusammenhang möglich, dass die Leitvorrichtung 100 einen Helligkeitssensor aufweist, der mit der Steuer- und Verarbeitungseinheit 4 verbunden ist. Vor einem solchen Helligkeitssensor kann sich optional ebenfalls ein optischer Filter befinden. Ab einer bestimmten Schwellenintensität des einfallenden Lichts, z.B. Infrarotlichts, kann das Signal von der Steuer- und Verarbeitungseinheit 4 gefiltert werden. Die Schwellenintensität bezeichnet dabei jene Intensität, ab der eine Trennung des Sensorlichts d.h. Nutzlichts des jeweiligen Lidar-Sensors 1a, 1b vom Störlicht nicht mehr garantiert werden kann, sodass die Brille u.U. nicht mehr 100% zuverlässig ist. Auf diese Weise kann effektiv vermieden werden, dass "zu viele" Hindernisse an die Person rückgemeldet werden, da über der Schwellenintensität auch Störlicht als fiktives Hindernis erfasst werden würde.

Dies kann gegebenenfalls auch der Person bzw. dem Träger der Leitvorrichtung 100 über ein entsprechendes Ausgabesignal mitgeteilt werden.

Des weiteren lassen sich kurzzeitig auftretende Signalspitzen über eine programmiertechnisch herbeigeführte Latenz softwaretechnisch von der Steuer- und Verarbeitungseinheit 4 ausblenden. Dabei kann über eine definierte Zeitdauer die durchschnittlich einfallende Signalstärke gemittelt werden und Signale, die überdurchschnittlich von diesem Wert abweichen, gefiltert werden. Dabei werden nur Hindernisse an die Person bzw. den Träger der Leitvorrichtung 100 rückgemeldet, wenn das von demselben Hindernis am jeweiligen Lidar-Sensor 1a, 1b eintreffende Messsignal über eine vorgegebene Zeitdauer vorhanden ist und somit über diese vorgegebene Zeitdauer derselbe Abstandsmesswert ermittelt wird. Auf diese Weise kann besonders effektiv vermieden werden, dass nur kurzzeitig erfasste Hindernisse im Gefahrenbereich der Person rückgemeldet werden, die auf Störlichtquellen oder anderen Störquellen der Umwelt (z.B. Staub oder Regen) zurückzuführen sind.

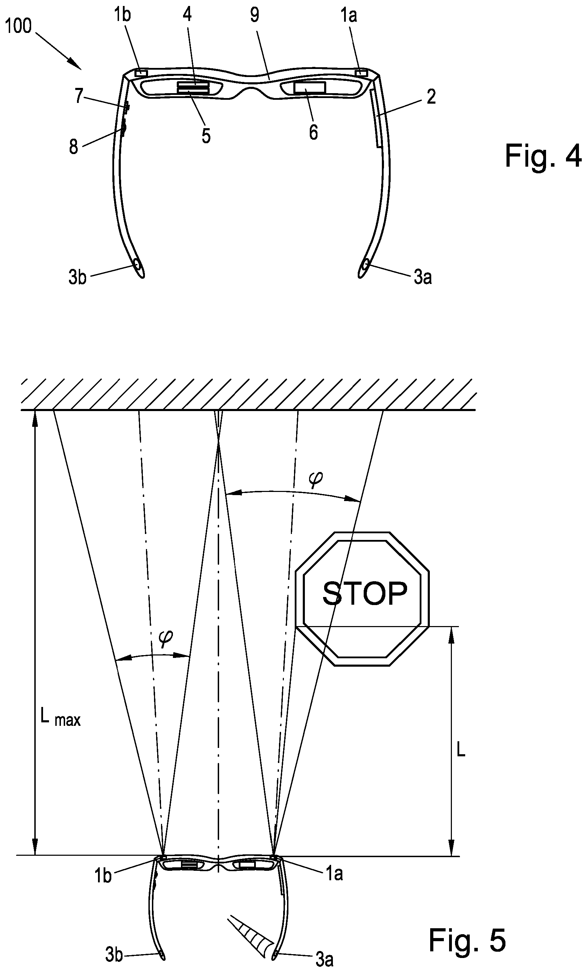

Fig. 5 zeigt nun ein schematisches Beispiel für die Funktionsweise einer erfindungsgemäßen Leitvorrichtung 100, wie sie im ersten Ausführungsbeispiel oder im zweiten Ausführungsbeispiel dargestellt ist.

Von den beiden Lidar-Sensoren 1a, 1b, die an der Frontseite der Brille 9 angeordnet sind, wird ein Laserstrahl ausgesendet. Jeder der Lidar-Sensoren 1a, 1b besitzt eine Öffnungswinkel φ, der in der Steuer- und Verarbeitungseinheit 4 hinterlegt ist und innerhalb dessen Objekte detektiert werden können. Die in Fig. 5 dargestellte Länge Lₘₐₓ steht für die maximale Distanz, bis zu der der Person, die die Leitvorrichtung 100 trägt, ein Hindernis gemeldet wird, d.h. die maximale Distanz, über die die Lidar-Sensoren 1a, 1b Messwerte für die Abstandsmessung empfangen. Diese Distanz kann beispielsweise bei zumindest 2,5 m liegen, was dem Träger der Leitvorrichtung 100 eine komfortable und sichere Fortbewegung bzw. Orientierung in seiner Umgebung ermöglicht. Diese Distanz kann durch entsprechende Auswahl der Lidar-Sensoren 1a, 1b festgelegt werden.

Wie in Fig. 5 ersichtlich ist, bewegt sich die Person, die die Leitvorrichtung 100 trägt, in einer Umgebung, in der ein Stop-Schild vorhanden ist, das sich in einer Entfernung L zur Person befindet. Trifft der jeweils von den Lidar-Sensoren 1a, 1b ausgesandte Laserstrahl nun auf ein Objekt bzw. Hindernis, wie das in Fig. 5 schematisch dargestellte Stop-Schild, wird von diesem Hindernis Licht in Richtung der Lidar-Sensoren 1a, 1b reflektiert und von den Lidar-Sensoren 1a, 1b detektiert.

Aufgrund der Laufzeit, die das reflektierte Licht benötigt, um vom Objekt, an dem es reflektiert wird, zum Lidar-Sensor 1a, 1b zu gelangen, wird die Entfernung L des Hindernisses zum Träger der Leitvorrichtung 100 berechnet. Wie im Ausführungsbeispiel in Fig. 5 ersichtlich ist, detektiert der rechte Lidar-Sensor 1a das Stop-Schild in einer Entfernung L von der Person. Die Steuer- und Verarbeitungseinheit 4 wandelt den ermittelten Abstandsmesswert in ein Steuersignal für die rechte Aktuator-Einheit 3a, die sich auf dem rechte Brillenbügel befindet, um. Dabei erstellt die Steuer- und Verarbeitungseinheit 4 das Steuersignal für die Aktuator-Einheiten 3a, 3b jeweils so, dass die Intensität des Signals zunimmt, wenn sich die Person an das Hindernis annähert.

Dies bedeutet im Ausführungsbeispiel konkret, dass je weiter sich die Person dem Stop-Schild nähert, d.h. je kleiner der Abstand zum Stop-Schild wird, umso stärker wird die Intensität z.B. der Vibration, die die Aktuator-Einheit 3a an die Person abgibt. Auf diese Weise wird dem Träger der Leitvorrichtung 100 nicht nur die Entfernung, sondern auch die Lage des Objekts über die integrierten Aktuator-Einheiten 3a, 3b übermittelt, da jeweils diejenige Aktuator-Einheit 3a, 3b ein Signal abgibt, die mit dem Lidar-Sensor 1a, 1b korrespondiert, der das Objekt detektiert hat. Wird ein Hindernis von beiden Lidar-Sensoren 1a, 1b detektiert, erstellt die Steuer- und Verarbeitungseinheit 4 auch dementsprechende Steuersignale für beide Aktuator-Einheiten 3a, 3b. Die Abgabe von Vibrationen ist im Ausführungsbeispiel in Form eines Signalkegels, der von der rechten Aktuator-Einheit 3a ausgeht, gekennzeichnet.

Optional kann die Steuer- und Verarbeitungseinheit 4 die ermittelten Abstandsmesswerte auch mit einem vorab vorgegebenen oder vom Träger der Leitvorrichtung 100 einstellbaren Abstandsschwellenwert vergleichen und auf Grundlage des Vergleichsergebnisses die Intensität des von der jeweiligen Aktuator-Einheit 3a, 3b abzugebenden Signals regulieren.

Optional kann die Intensität hierbei auch 0 betragen, wenn der jeweils ermittelte Abstandsmesswert über dem vorab vorgegebenen und/oder eingestellten Abstandsschwellenwert liegt. Dies kann besonders vorteilhaft sein, wenn sich eine Person mit der Leitvorrichtung 100 beispielsweise in einem Stadtgebiet bewegt, wo eine Vielzahl von Hindernissen in einem kurzen Distanzbereich vorhanden sind. In diesem Fall würde die Person laufend Warnsignale über die Aktuator-Einheiten 3a, 3b empfangen, obwohl sich vielleicht einige Hindernisse erst in vergleichsweise größerer Entfernung befinden und die Person noch nicht unmittelbar gefährden. Liegt der ermittelte Abstandsmesswert über dem Abstandsschwellenwert, wird kein Signal an die Person bzw. den Träger der Leitvorrichtung 100 abgegeben.

Um hier nur vor Hindernissen gewarnt zu werden, die die Person unmittelbar gefährden, da sie sich in relativ kurzer Distanz befinden, kann die Leitvorrichtung 100 optional wie im ersten und im zweiten Ausführungsbeispiel einen Schalter 8 umfassen, der verschiedene Schaltpositionen aufweist, die die Person bzw. der Träger der Leitvorrichtung 100 frei wählen kann. Diese Schaltpositionen sind jeweils mit einem Abstandsschwellenwert assoziiert, der von der Steuer- und Verarbeitungseinheit 4 für den Vergleich mit den ermittelten Abstandsmesswerten herangezogen werden soll. Hierzu können beispielsweise folgende, in Tabelle 1 zusammengefasste, drei Einstellungen für Abstandsschwellenwerte bzw. Schaltpositionen vorgegeben sein:

**Tabelle 1: Beispiele für wählbare Abstandsschwellenwerte bzw. Schalterpositionen**

| Distanz | Umgebung |
|---|---|
| 0,8 Meter | Innen-Modus: dabei kann die Distanz klein gehalten werden um die Leitvorrichtung 100 z.B. in einem Gebäude zu verwenden |
| 1,5 Meter | Außen-Modus belebt: diese Distanz kann z.B. in einer belebten Umgebung angewendet werden (z.B. belebte Einkaufsstraßen) |
| 2,5 Meter | Außen-Modus: dieser Modus kann im Freien angewendet werden, wenn z.B. keine oder wenige Personen oder Verkehrsmittel in der Umgebung des Benutzers vorhanden sind (z.B. Wege und Straßen im ländlichen Raum) |

Optional ist es selbstverständlich auch möglich, dass die Steuer- und Verarbeitungseinheit 4 aus einer Reihe von hinterlegten Abstandsschwellenwerten einen an die aktuelle Umgebungssituation der Person, die die Leitvorrichtung 100 trägt, angepassten Abstandsschwellenwert auswählt. Dazu kann die Steuer- und Verarbeitungseinheit 4 die Anzahl derjenigen ermittelten Abstandsmesswerte, die den aktuellen Abstandsschwellenwert unterschreiten, ermitteln. Wenn diese ermittelte Anzahl einen vorgegebenen Anzahlschwellenwert überschreitet, kann die Steuer- und Verarbeitungseinheit 4 dann den nächstniedrigeren Abstandsschwellenwert, d.h. denjenigen Abstandsschwellenwert, der sich in nächst kürzerer Distanz zur Person befindet, auswählen und diesen dem Nutzer optional durch ein entsprechendes Signal rückmelden.

Da sämtliche Elektronik einer erfindungsgemäßen Leitvorrichtung 100 in bzw. an der Brille 9 untergebracht ist, ist diese besonders kompakt und weist nur geringes Gewicht auf. Sämtliche elektrische Schaltkreise und Leiterplatten können hierzu auf individuellen Platinen zusammengeführt sein. Dies hat zum einen den Vorteil, sehr wenige Kabel zu benötigen, da sämtliche Verbindungen intern auf einer Leiterplatte integriert sind. Zum anderen lässt sich dadurch auch der benötigte Platzbedarf erheblich minimieren. Es wird daher möglich, die Technik unscheinbar hinter dem Brillenglas bzw. auf der Innenseite des Brillenrahmens oder Brillenbügels zu integrieren.

Eine erfindungsgemäße Leitvorrichtung 100 ist somit einerseits besonders kompakt aufgebaut und stellt somit selbst bei einem ganztägigen Gebrauch keine Einschränkung und Belastung für den Benutzer bzw. den Träger dar, da sie sich in Größe und Gewicht kaum von einer handelsüblichen Brille bzw. Sonnenbrille unterscheidet. Andererseits ist die Leitvorrichtung 100 für außenstehende Personen nicht von einer solchen handelsüblichen Brille bzw. Sonnenbrille zu unterscheiden.

Selbstverständlich kann eine erfindungsgemäße Leitvorrichtung 100 optional auch eine Energieversorgungseinheit 2 umfassen, z.B. einen leistungsstarken Akku, der die Leitvorrichtung 100 einen Arbeitstag lang, z.B. 12h lang, mit Energie versorgt. Eine derartige Energieversorgungseinheit 2 kann, wie im ersten und im zweiten Ausführungsbeispiel, z.B. an der Innenseite eines Brillenbügels angeordnet sein. Optional kann sich die Energieversorgungseinheit 2 auch z.B. an der Innenseite eines Brillenglases befinden.

Eine erfindungsgemäße Leitvorrichtung 100 kann optional auch noch eine oder mehrere der folgenden Komponenten umfassen, wie dies in variierender Kombination im ersten und im zweiten Ausführungsbeispiel dargestellt ist:
Optional kann eine erfindungsgemäße Leitvorrichtung 100 ein Lademodul 5 und/oder ein Kommunikationsmodul 6 aufweisen. Diese können optional auf einer einzigen Platine, gegebenenfalls mit der Steuer- und Verarbeitungseinheit 4 zusammengeführt sein und stehen mit der Steuer- und Verarbeitungseinheit 4 in Verbindung.

Ein derartiges Lademodul 5 kann als Ladestands- bzw. Batteriemanagementsystem der Leitvorrichtung 100 fungieren. Mittels eines derartigen Lademoduls 5kann eine in der Leitvorrichtung 100 integrierte Energieversorgungseinheit 2 bzw. ein Akku ge- und entladen werden. Das Lademodul 5 kann dabei in der Platine mit anderen elektrischen Schaltkreisen integriert sein. Der Ladeanschluss kann an einer geeigneten Stelle in der Brille integriert sein, wie z.B. an einem Ende eines Brillenbügels oder seitlich hinter einem Brillenglas.. Der Ladeanschluss kann dabei in Form einer USB-Buchse ausgeführt sein, oder auch über ein individuelles Kontaktkabel erfolgen. Anstelle eines Ladeanschlusses kann auch eine Induktionsspule in die Leitvorrichtung 100 integriert werden, z.B. hinter einem der Brillengläser, um eine Energieversorgungseinheit 2 bzw. einen Akku der Leitvorrichtung 100 kontaktlos laden zu können, z.B. gemäß QI - Standard.

Ein derartiges Kommunikationsmodul 6 kann als Kommunikationsschnittstelle der Steuer- und Verarbeitungseinheit 4 eingesetzt werden. Mittels eines derartigen Kommunikationsmoduls 6 kann zu verschiedensten Zwecken mit der Software der Steuer- und Verarbeitungseinheit 4 kommuniziert werden. Damit wird es einerseits möglich, die Software zu aktualisieren, und andererseits mögliche Fehler bzw. Fehlermeldungen auszulesen. In weitere Folge können auch gesammelte Daten der Steuer- und Verarbeitungseinheit 4 ausgelesen werden, um diese für verschiedenste Anwendungen, wie z.B. zur Erstellung einer Hindernisdatenbank oder zur Sensoroptimierung, zu verwenden. Zur Herstellung der Kommunikationsverbindung kann das Kommunikationsmodul dabei über einen eigenen Anschluss, z.B. eine USB-Buchse oder ein individuelles Kontaktkabel, verfügen, wie dieser bereits für das Lademodul 5 beschrieben wurde. Zusätzlich oder alternativ dazu kann eine Kommunikationsverbindung z.B. über einen kontaktlosen Kommunikationsstandard wie z.B. Bluetooth oder W-LAN hergestellt werden.

Optional kann bei einer Kombination eines Lademoduls 5 mit einem Kommunikationsmodul 6 ein am Lademodul 5 vorhandener Anschluss (z.B. USB-Buchse oder individuelles Kontaktkabel), für die Herstellung der Kommunikationsverbindung mit dem Kommunikationsmodul 6 herangezogen werden.

Optional kann eine erfindungsgemäße Leitvorrichtung 100 einen Hauptschalter 7 umfassen. Ein derartiger Hauptschalter 7 kann dazu dienen, die Leitvorrichtung 100 ein- und auszuschalten, um auf diese Weise den Energieverbrauch der Leitvorrichtung 100 zu reduzieren. Optional kann dem Benutzer sowohl beim Ein-, als auch beim Ausschalten über ein charakteristisches Feedback-Signal die jeweilige Aktion rückgemeldet werden.

## Patentansprüche

1. Leitvorrichtung (100) für Personen mit eingeschränktem Sehvermögen umfassend eine Brille (9), zwei Lidar-Sensoren (1a, 1b), zwei Aktuator-Einheiten (3a, 3b) und eine mit den Lidar-Sensoren (1a, 1b) und den Aktuator-Einheiten (3a, 3b) verbundene Steuer- und Verarbeitungseinheit (4),
- wobei die Lidar-Sensoren (1a, 1b) an der Frontseite der Brille (9), insbesondere am Brillenrahmen, angeordnet sind und zur Erfassung der Umgebung des jeweiligen Lidar-Sensors (1a, 1b), insbesondere des Abstands zu Hindernissen, ausgebildet sind, wobei ein Filter zur Reduktion des Einflusses von Störlicht auf die Messwerte der Lidar-Sensoren (1a, 1b) vorgesehen ist,
- wobei die Steuer- und Verarbeitungseinheit (4) an der, dem Kopf einer Person zugewandten, Innenseite der Brille (9), insbesondere am Brillenrahmen, am Brillenglas oder an einem Brillenbügel, angeordnet ist und dazu ausgebildet ist, Abstandsmessungen mittels der Lidar-Sensoren (1a, 1b) durchzuführen und die ermittelten Abstandsmesswerte in Steuersignale für die Aktuator-Einheiten (3a, 3b) umzuwandeln und an die Aktuator-Einheiten (3a, 3b) zu übermitteln, und
- wobei jede Aktuator-Einheit (3a, 3b) jeweils an der Innenseite eines Brillenbügels angeordnet ist und dazu ausgebildet ist, zur Darstellung der Abstandsmesswerte für Personen wahrnehmbare Signale abzugeben,
- wobei die Leitvorrichtung (100) zumindest einen, insbesondere am Brillenbügel angeordneten, mit der Steuer- und Verarbeitungseinheit (4) verbundenen, Schalter (8) umfasst, wobei der Schalter (8) zumindest eine, mit einem, insbesondere in der Steuer- und Verarbeitungseinheit (4) hinterlegten, Abstandsschwellenwert assoziierte, Schaltposition, vorzugsweise mehrere Schaltpositionen, aufweist,
- wobei die Steuer- und Verarbeitungseinheit (4) dazu ausgebildet ist, bei Verstellen des Schalters (8) in eine Schaltposition den mit der jeweiligen Schaltposition assoziierten Abstandsschwellenwert für die Umwandlung der ermittelten Abstandsmesswerte in Steuersignale für die Aktuator-Einheiten (3a, 3b), insbesondere für die Vorgabe der Intensität des von der jeweiligen Aktuator-Einheit (3a, 3b) abzugebenden Signals, heranzuziehen.

2. Leitvorrichtung (100) nach Anspruch 1, **dadurch gekennzeichnet, dass** vor jedem der Lidar-Sensoren (1a, 1b) jeweils ein optischer Filter angeordnet ist, wobei der optische Filter dazu ausgebildet ist, Licht mit einer Wellenlänge von 780 nm bis 1 mm, insbesondere 780 nm bis 3 µm, vorzugsweise 940 nm, passieren zu lassen.

3. Leitvorrichtung (100) nach Anspruch 1 oder 2, **dadurch gekennzeichnet, dass** die Steuer- und Verarbeitungseinheit (4) dazu ausgebildet ist, ausgewählte, vorzugsweise alle, ermittelten Abstandsmesswerte jeweils einem digitalen Filter zur Reduktion des Einflusses von Störlicht zuzuführen und die derartigen durch Filterung ermittelten gefilterten Abstandsmesswerte für die Umwandlung in Steuersignale heranzuziehen.

4. Leitvorrichtung (100) nach einem der vorangehenden Ansprüche, **dadurch gekennzeichnet,**
- **dass** ein Lidar-Sensor (1a) rechts und ein weiterer Lidar-Sensor (1b) links jeweils an der Frontseite der Brille (9), insbesondere am Brillenrahmen, angeordnet ist, und
- **dass** eine Aktuator-Einheit (3a) am linken Brillenbügel und eine weitere Aktuator-Einheit (3b) am rechten Brillenbügel angeordnet ist.

5. Leitvorrichtung (100) nach Anspruch 4, **dadurch gekennzeichnet, dass** die Steuer- und Verarbeitungseinheit (4) dazu ausgebildet ist, die vom linken Lidar-Sensor (1a) stammenden Abstandsmesswerte in Steuersignale für die linke Aktuator-Einheit (3a) umzuwandeln und die vom rechten Lidar-Sensor (1b) stammenden Abstandsmesswerte in Steuersignale für die rechte Aktuator-Einheit (3b) umzuwandeln.

6. Leitvorrichtung (100) nach einem der vorangehenden Ansprüche, **dadurch gekennzeichnet, dass** die Steuer- und Verarbeitungseinheit (4) dazu ausgebildet ist, die Intensität des von der jeweiligen Aktuator-Einheit (3a, 3b) abzugebenden Signals in Abhängigkeit vom ermittelten Abstandsmesswert vorzugeben, insbesondere die Intensität des Signals bei sich verringerndem Abstand zu erhöhen.

7. Leitvorrichtung (100) nach Anspruch 6, **dadurch gekennzeichnet, dass** die Steuer- und Verarbeitungseinheit (4) dazu ausgebildet ist, den jeweils ermittelten Abstandsmesswert mit zumindest einem, insbesondere vorab vorgegebenen und/oder einstellbaren, Abstandsschwellenwert zu vergleichen und auf Grundlage des Vergleichs die Intensität des von der jeweiligen Aktuator-Einheit (3a, 3b) abzugebenden Signals vorzugeben.

8. Leitvorrichtung (100) nach einem der vorangehenden Ansprüche, **dadurch gekennzeichnet, dass** die Aktuator-Einheiten (3a, 3b) als Akustikelemente, insbesondere in Form von Gehörknochen-Lautsprechern, und/oder taktile Elemente ausgebildet sind.

9. Leitvorrichtung (100) nach einem der vorangehenden Ansprüche, **dadurch gekennzeichnet, dass** die Leitvorrichtung (100) eine an der, dem Kopf einer Person zugewandten, Innenseite der Brille (9), insbesondere am Brillenrahmen oder an einem Brillenbügel, angeordnete Energieversorgungseinheit (2) umfasst,
wobei insbesondere vorgesehen ist, dass die Steuer- und Verarbeitungseinheit (4) dazu ausgebildet, ist, den Ladestand der Energieversorgungseinheit (2), insbesondere laufend und/oder in vorgegebenen Zeitabständen, zu überprüfen und, im Fall, dass der Ladestand einen vorgegebenen Ladestand-Schwellenwert unterschreitet, ein diesbezügliches Steuersignal für zumindest eine Aktuator-Einheit (3a, 3b) zu erstellen und an die Aktuator-Einheiten (3a, 3b) zu übermitteln.

## Claims

1. Guidance device (100) for visually impaired persons comprising a pair of glasses (9), two lidar sensors (1a, 1b), two actuator units (3a, 3b) and a control and processing unit (4) connected to the lidar sensors (1a, 1b) and the actuator units (3a, 3b),
- wherein the lidar sensors (1a, 1b) are arranged on the front side of the glasses (9), in particular on the frame of the glasses, and are configured to detect the environment of the respective lidar sensor (1a, 1b), in particular the distance to obstacles, wherein a filter for reducing the influence of stray light on the measurement values of the lidar sensors (1a, 1b) is provided,
- wherein the control and processing unit (4) is arranged on the inner side of the glasses (9) facing the head of a person, in particular on the frame of the glasses, on the spectacle lens or on a temple of the glasses, and is configured to perform distance measurements by means of the lidar sensors (1a, 1b) and to convert the determined distance measurement values into control signals for the actuator units (3a, 3b) and to transmit them to the actuator units (3a, 3b), and
- wherein each actuator unit (3a, 3b) is arranged on the inner side of a temple of the glasses and is configured to emit signals perceptible to persons for representing the distance measurement values,
- wherein the guidance device (100) comprises at least one switch (8), in particular arranged on the temple of the glasses, connected to the control and processing unit (4), wherein the switch (8) has at least one switching position, preferably several switching positions, associated with a distance threshold value, in particular stored in the control and processing unit (4),
- wherein the control and processing unit (4) is configured, when the switch (8) is adjusted into a switching position, to use the distance threshold value associated with the respective switching position for converting the determined distance measurement values into control signals for the actuator units (3a, 3b), in particular for specifying the intensity of the signal to be emitted by the respective actuator unit (3a, 3b).

2. Guidance device (100) according to claim 1, **characterized in that** an optical filter is arranged in front of each of the lidar sensors (1a, 1b), wherein the optical filter is configured to allow light with a wavelength of 780 nm to 1 mm, in particular 780 nm to 3 µm, preferably 940 nm, to pass through.

3. Guidance device (100) according to claim 1 or 2, **characterized in that** the control and processing unit (4) is configured to feed selected, preferably all, determined distance measurement values to a respective digital filter for reducing the influence of stray light and to use the filtered distance measurement values determined in this way by filtering for conversion into control signals.

4. Guidance device (100) according to any of the preceding claims, **characterized in that**
- a lidar sensor (1a) is arranged on the right and another lidar sensor (1b) is arranged on the left on the front side of the glasses (9), in particular on the frame of the glasses, and
- an actuator unit (3a) is arranged on the left temple and another actuator unit (3b) is arranged on the right temple of the glasses.

5. Guidance device (100) according to claim 4, **characterized in that** the control and processing unit (4) is configured to convert the distance measurement values originating from the left lidar sensor (1a) into control signals for the left actuator unit (3a) and to convert the distance measurement values originating from the right lidar sensor (1b) into control signals for the right actuator unit (3b).

6. Guidance device (100) according to any of the preceding claims, **characterized in that** the control and processing unit (4) is configured to specify the intensity of the signal to be emitted by the respective actuator unit (3a, 3b) as a function of the determined distance measurement value, in particular to increase the intensity of the signal as the distance decreases.

7. Guidance device (100) according to claim 6, **characterized in that** the control and processing unit (4) is configured to compare the respectively determined distance measurement value with at least one distance threshold value, which is in particular predetermined and/or adjustable, and to specify the intensity of the signal to be emitted by the respective actuator unit (3a, 3b) on the basis of the comparison.

8. Guidance device (100) according to any of the preceding claims, **characterized in that** the actuator units (3a, 3b) are configured as acoustic elements, in particular in the form of bone conduction speakers, and/or tactile elements.

9. Guidance device (100) according to any of the preceding claims, **characterized in that** the guidance device (100) comprises a power supply unit (2) arranged on the inner side of the glasses (9) facing the head of a person, in particular on the frame of the glasses or on a temple of the glasses,
wherein in particular it is provided that the control and processing unit (4) is configured to monitor the charge state of the power supply unit (2), in particular continuously and/or at predetermined time intervals, and, in the event that the charge state falls below a predetermined charge state threshold value, to generate a corresponding control signal for at least one actuator unit (3a, 3b) and to transmit it to the actuator units (3a, 3b).

## Revendications

1. Dispositif de guidage (100) pour personnes à vision réduite, comprenant des lunettes (9), deux capteurs Lidar (1a, 1b), deux unités d'actionneur (3a, 3b) et une unité de commande et de traitement (4) reliée aux capteurs Lidar (1a, 1b) et aux unités d'actionneur (3a, 3b),
- dans lequel les capteurs Lidar (1a, 1b) sont disposés sur la face avant des lunettes (9), en particulier sur la monture des lunettes, et sont configurés pour détecter l'environnement du capteur Lidar (1a, 1b) respectif, en particulier la distance par rapport à des obstacles, un filtre étant prévu pour réduire l'influence de la lumière parasite sur les valeurs de mesure des capteurs Lidar (1a, 1b),
- dans lequel l'unité de commande et de traitement (4) est disposée sur la face interne des lunettes (9) tournée vers la tête d'une personne, en particulier sur la monture des lunettes, sur le verre des lunettes ou sur une branche des lunettes, et est configurée pour effectuer des mesures de distance au moyen des capteurs Lidar (1a, 1b) et pour convertir les valeurs de mesure de distance déterminées en signaux de commande pour les unités d'actionneur (3a, 3b) et les transmettre aux unités d'actionneur (3a, 3b), et
- dans lequel chaque unité d'actionneur (3a, 3b) est disposée sur la face interne d'une branche des lunettes et est configurée pour émettre des signaux perceptibles par des personnes pour représenter les valeurs de mesure de distance,
- dans lequel le dispositif de guidage (100) comprend au moins un commutateur (8), disposé en particulier sur la branche des lunettes, relié à l'unité de commande et de traitement (4), le commutateur (8) présentant au moins une position de commutation, de préférence plusieurs positions de commutation, associée à une valeur seuil de distance, en particulier enregistrée dans l'unité de commande et de traitement (4),
- dans lequel l'unité de commande et de traitement (4) est configurée pour, lors du déplacement du commutateur (8) dans une position de commutation, utiliser la valeur seuil de distance associée à la position de commutation respective pour convertir les valeurs de mesure de distance déterminées en signaux de commande pour les unités d'actionneur (3a, 3b), en particulier pour spécifier l'intensité du signal devant être émis par l'unité d'actionneur (3a, 3b) respective.

2. Dispositif de guidage (100) selon la revendication 1, **caractérisé en ce qu'**un filtre optique est disposé devant chacun des capteurs Lidar (1a, 1b), le filtre optique étant configuré pour laisser passer la lumière ayant une longueur d'onde de 780 nm à 1 mm, en particulier de 780 nm à 3 µm, de préférence de 940 nm.

3. Dispositif de guidage (100) selon la revendication 1 ou 2, **caractérisé en ce que** l'unité de commande et de traitement (4) est configurée pour acheminer des valeurs de mesure de distance déterminées sélectionnées, de préférence toutes, respectivement vers un filtre numérique pour réduire l'influence de la lumière parasite et pour utiliser les valeurs de mesure de distance filtrées ainsi déterminées par filtrage pour la conversion en signaux de commande.

4. Dispositif de guidage (100) selon l'une des revendications précédentes, **caractérisé en ce que** :
- un capteur Lidar (1a) est disposé à droite et un autre capteur Lidar (1b) est disposé à gauche, respectivement sur la face avant des lunettes (9), en particulier sur la monture des lunettes, et
- une unité d'actionneur (3a) est disposée sur la branche gauche des lunettes et une autre unité d'actionneur (3b) est disposée sur la branche droite des lunettes.

5. Dispositif de guidage (100) selon la revendication 4, **caractérisé en ce que** l'unité de commande et de traitement (4) est configurée pour convertir les valeurs de mesure de distance provenant du capteur Lidar gauche (1a) en signaux de commande pour l'unité d'actionneur gauche (3a) et pour convertir les valeurs de mesure de distance provenant du capteur Lidar droit (1b) en signaux de commande pour l'unité d'actionneur droite (3b).

6. Dispositif de guidage (100) selon l'une des revendications précédentes, **caractérisé en ce que** l'unité de commande et de traitement (4) est configurée pour spécifier l'intensité du signal devant être émis par l'unité d'actionneur (3a, 3b) respective en fonction de la valeur de mesure de distance déterminée, en particulier pour augmenter l'intensité du signal lorsque la distance diminue.

7. Dispositif de guidage (100) selon la revendication 6, **caractérisé en ce que** l'unité de commande et de traitement (4) est configurée pour comparer la valeur de mesure de distance respectivement déterminée avec au moins une valeur seuil de distance, qui est en particulier prédéfinie et/ou réglable, et pour spécifier l'intensité du signal devant être émis par l'unité d'actionneur (3a, 3b) respective sur la base de la comparaison.

8. Dispositif de guidage (100) selon l'une des revendications précédentes, **caractérisé en ce que** les unités d'actionneur (3a, 3b) sont configurées comme des éléments acoustiques, en particulier sous la forme de haut-parleurs à conduction osseuse, et/ou des éléments tactiles.

9. Dispositif de guidage (100) selon l'une des revendications précédentes, **caractérisé en ce que** le dispositif de guidage (100) comprend une unité d'alimentation en énergie (2) disposée sur la face interne des lunettes (9) tournée vers la tête d'une personne, en particulier sur la monture des lunettes ou sur une branche des lunettes,
dans lequel il est en particulier prévu que l'unité de commande et de traitement (4) est configurée pour surveiller l'état de charge de l'unité d'alimentation en énergie (2), en particulier en continu et/ou à des intervalles de temps prédéfinis, et, au cas où l'état de charge est inférieur à une valeur seuil d'état de charge prédéfinie, pour générer un signal de commande correspondant pour au moins une unité d'actionneur (3a, 3b) et le transmettre aux unités d'actionneur (3a, 3b).
